(19) Europäisches Patentamt / European Patent Office / Office européen des brevets

(11) **EP 4 422 943 B1**

(12) **EUROPÄISCHE PATENTSCHRIFT**

(45) Veröffentlichungstag und Bekanntmachung des Hinweises auf die Patenterteilung:
**24.09.2025   Patentblatt 2025/39**

(21) Anmeldenummer: **22798101.6**

(22) Anmeldetag: **30.09.2022**

(51) Internationale Patentklassifikation (IPC):
*B60W 30/18* (2012.01)        *B60W 40/09* (2012.01)
*B60W 50/14* (2020.01)        *B60W 50/00* (2006.01)

(52) Gemeinsame Patentklassifikation (CPC):
**B60W 30/18163; B60W 40/09; B60W 50/14;**
B60W 2050/0088; B60W 2540/20; B60W 2540/223;
B60W 2540/229

(86) Internationale Anmeldenummer:
**PCT/EP2022/077269**

(87) Internationale Veröffentlichungsnummer:
**WO 2023/072524 (04.05.2023 Gazette 2023/18)**

(54) **VERFAHREN ZUM UNTERSTÜTZEN EINES NUTZERS EINES FAHRZEUGS BEIM MANÖVRIEREN DES FAHRZEUGS AUF EINER MEHRSPURIGEN STRASSE UNTER BERÜCKSICHTIGUNG EINER INDIVIDUELLEN REAKTIONSZEIT DES NUTZERS, FAHRERASSISTENZSYSTEM SOWIE FAHRZEUG**

METHOD FOR ASSISTING A VEHICLE USER DURING A MANOEUVRE OF THE VEHICLE ON A MULTI-LANE ROAD TAKING INTO ACCOUNT AN INDIVIDUAL REACTION TIME OF THE USER, DRIVER ASSISTANCE SYSTEM, AND VEHICLE

PROCÉDÉ D'ASSISTANCE À UN UTILISATEUR DE VÉHICULE PENDANT UNE MAN¿UVRE DU VÉHICULE SUR UNE ROUTE À PLUSIEURS VOIES EN TENANT COMPTE D'UN TEMPS DE RÉACTION INDIVIDUEL DE L'UTILISATEUR, SYSTÈME D'ASSISTANCE À LA CONDUITE ET VÉHICULE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **29.10.2021   DE 102021128328**

(43) Veröffentlichungstag der Anmeldung:
**04.09.2024   Patentblatt 2024/36**

(73) Patentinhaber: **Bayerische Motoren Werke Aktiengesellschaft**
**80809 München (DE)**

(72) Erfinder:
• **NIERMANN, Stephan**
  **85241 Hebertshausen (DE)**
• **RIETH, Dominik**
  **80992 München (DE)**
• **FAYAD, Sami**
  **81369 München (DE)**

(56) Entgegenhaltungen:
DE-A1- 102014 219 110     DE-A1- 102016 216 134
DE-A1- 102016 216 135     DE-A1- 102019 008 318
US-A1- 2017 308 090

**Beschreibung**

Die vorliegende Erfindung betrifft ein Verfahren zum Unterstützen eines Nutzers eines Fahrzeugs beim Manövrieren des Fahrzeugs auf einer mehrspurigen Straße. Darüber hinaus betrifft die vorliegende Erfindung ein Fahrerassistenzsystem. Schließlich betrifft die vorliegende Erfindung ein Fahrzeug mit einem derartigen Fahrerassistenzsystem.

Aus dem Stand der Technik sind Fahrerassistenzsysteme zum Durchführen von zumindest teilautomatisierten Spurwechselmanövern bzw. so genannte Spurwechselassistenzsysteme bekannt. Bei diesen Fahrerassistenzsystemen mit automatisierter bzw. automatischer Spurwechselfunktion zeigt der Nutzer bzw. Fahrer typischerweise durch eine bestimmte Bedienhandlung einen Spurwechsel an. Wenn eine solche Bedienhandlung erkannt wird, wird mittels des Fahrerassistenzsystems das Fahrzeug entlang einer geplanten Trajektorie auf einen benachbarten Fahrstreifen bzw. die Zielspur mit automatischer Querführung und im Allgemeinen auch mit automatischer Längsführung manövriert. Bei derartigen Fahrerassistenzsystemen bzw. Spurwechselassistenzsystemen wird üblicherweise der fahrerseitige Spurwechselwunsch durch das Betätigen eines entsprechenden Bedienelements, beispielsweise eines Blinkerhebels, zum Aktivieren von außerhalb des Fahrzeugs sichtbaren Fahrtrichtungsanzeigern signalisiert.

Bekannte Fahrerassistenzsysteme bzw. Spurwechselassistenzsysteme überwachen im Allgemeinen vor dem Spurwechsel mittels einer geeigneten Umfeldsensorik das Umfeld des Fahrzeugs. Auf die Bedienhandlung des Fahrers hin kann dann der Fahrstreifen gewechselt werden, falls auf dem benachbarten Fahrstreifen eine freie Lücke für das eigene Fahrzeug erkannt wurde.

Des Weiteren sind aus dem Stand der Technik Fahrerassistenzsysteme bekannt, welche den Nutzer des Fahrzeugs auf einer mehrspurigen Straße bei einem Erreichen einer Ausfahrt unterstützen. Dabei kann das Fahrerassistenzsystem beispielsweise infolge einer Eingabe von einem Navigationsziel alle nötigen Spurwechsel bis zum Erreichen der Ausfahrt vorbereiten. Hierzu kann das Fahrerassistenzsystem zunächst eine freie Lücke für das Fahrzeug auf der Zielspur erkennen, und anschließend die Geschwindigkeit des Fahrzeugs für das nachfolgende Spurwechselmanöver in die erkannte Lücke anpassen. Der Spurwechsel selbst kann dann mittels eines Spurwechselassistenzsystems durchgeführt werden, wobei das Spurwechselassistenzsystem automatisch oder nach einer Bedieneingabe durch den Nutzer ausgelöst wird.

Wenn beispielsweise auf dem benachbarten Fahrstreifen bzw. der Zielspur eine freie Lücke für das eigene Fahrzeug erkannt wurde und/oder die Geschwindigkeit des Fahrzeugs für das nachfolgende Spurwechselmanöver entsprechend angepasst wurde, wird an den Nutzer ein entsprechender Hinweis zum Auslösen des Spurwechselmanövers ausgegeben. Infolge dieses Hinweises kann dann der Nutzer ein entsprechendes Bedienelement, beispielsweise den Blinkerhebel, betätigen und somit das zumindest teilautomatisierte Spurwechselmanöver auslösen. Gemäß dem Stand der Technik wird dieser Spurwechselhinweis zu einem zuvor definierten, festen Zeitpunkt ausgegeben.

In diesem Zusammenhang offenbart die DE 10 2016 216 135 A1 ein Assistenzsystem, welches eingerichtet ist, aufgrund von Information eines Navigationssystems zur Zielführung festzustellen, dass mehrere Spurwechsel ausgehend von der aktuellen Fahrspur auf eine Zielfahrspur erforderlich sind. Falls mittels des Assistenzsystems festgestellt wird, dass eine Auslöse-Bedienhandlung des Fahrers vorliegt, werden die erforderlichen mehreren Spurwechsel ausgehend von der aktuellen Fahrspur auf eine Zielfahrspur mit zumindest automatisierter Querführung automatisiert seitens des Spurwechselassistenzsystems durchgeführt.

Die DE 10 2016 216 134 A1 zeigt ein Assistenzsystem für ein Kraftfahrzeug. Wenn mittels des Assistenzsystems festgestellt wurde, dass ein Spurwechsel erforderlich ist, wird der Fahrer über den erforderlichen Spurwechsel mit dem Ziel informiert, dass dieser den erforderlichen Spurwechsel einleitet. Hierzu wird die Querführung seitens des Fahrerassistenzsystems derart angepasst, dass die Querposition des Fahrzeugs in Bezug auf die aktuelle Fahrspur in Richtung derjenigen Seite der beiden Seiten der aktuellen Fahrspur verschoben wird, über die der erforderliche Spurwechsel zu erfolgen hat.

Außerdem beschreibt die DE 10 2019 008 318 A1 ein Verfahren zur Steuerung zumindest eines Fahrerassistenzsystems eines Fahrzeuges in Abhängigkeit einer körperlichen Verfassung eines Fahrzeugnutzers. Dabei ist vorgesehen, dass eine individuelle Reaktionszeit des Fahrzeugnutzers ermittelt wird, welche dem zumindest einen Fahrerassistenzsystem zur Einstellung einer Vorwarnzeit zur Erhöhung einer Aufmerksamkeit des Fahrzeugnutzers zugeführt wird.

Es ist Aufgabe der vorliegenden Erfindung, eine Lösung aufzuzeigen, wie ein Betrieb eines Fahrerassistenzsystems der eingangs genannten Art an den Nutzer angepasst werden kann. Darüber hinaus soll ein Fahrzeug mit einem entsprechenden Fahrerassistenzsystem bereitgestellt werden.

Diese Aufgabe wird erfindungsgemäß durch ein Verfahren, durch ein Fahrerassistenzsystem sowie durch ein Fahrzeug mit den Merkmalen gemäß den unabhängigen Ansprüchen gelöst. Vorteilhafte Weiterbildungen der vorliegenden Erfindung sind in den abhängigen Ansprüchen angegeben.

Ein erfindungsgemäßes Verfahren dient zum Unterstützen eines Nutzers eines Fahrzeugs beim Manövrieren des Fahrzeugs auf einer mehrspurigen Straße. Das Verfahren umfasst das Empfangen eines Fahrbefehls zum Manövrieren des Fahrzeugs von einem zweiten Fahrstreifen der Straße über einen ersten Fahrstreifen der Straße auf eine Ausfahrt der Straße. Des Weiteren umfasst das Verfahren das Suchen einer freien Lücke für das Fahrzeug zwischen Verkehrsteilnehmern auf dem ersten Fahrstreifen. Zudem umfasst das Verfahren das Ausgeben eines Hinweises an den Nutzer zum Betätigen eines Bedienelements zum Auslösen eines Spurwechselmanövers von dem zweiten Fahrstreifen in die freie Lücke des ersten Fahrstreifens, wobei der Hinweis zu einem bestimmten Hinweiszeitpunkt ausgegeben wird. Außerdem umfasst das Verfahren das Bestimmen einer Gesamtreaktionszeit, welche eine Zeitdauer zwischen dem Ausgeben des Hinweises und dem Auslösen des Spurwechselmanövers beschreibt. Zudem umfasst das Verfahren das Bestimmen eines individuellen Reaktionsfaktors des Nutzers anhand der Gesamtreaktionszeit sowie das Anpassen des Hinweiszeitpunkts für ein nachfolgendes Manövrieren des Fahrzeugs in Abhängigkeit von dem individuellen Reaktionsfaktor.

Mit Hilfe des Verfahrens soll der Nutzer bzw. ein Fahrer des Fahrzeugs beim Manövrieren des Fahrzeugs auf der mehrspurigen Straße unterstützt werden. Bei der mehrspurigen Straße kann es sich grundsätzlich um eine Fernstraße, eine Bundesstraße, eine Schnellstraße, eine autobahnähnliche Straße oder dergleichen handeln. Bevorzugt handelt es sich bei der mehrspurigen Straße aber um eine Autobahn. Die Straße kann beispielsweise zwei Richtungsfahrbahnen mit jeweils zumindest zwei Fahrstreifen, welche vorliegend als erster Fahrstreifen und zweiter Fahrstreifen bezeichnet werden, aufweisen. Auf diesen Fahrstreifen können sich sowohl das eigene Fahrzeug als auch weitere Verkehrsteilnehmer in eine vorgegebene Fahrtrichtung bewegen. Die Straße kann auch weitere Fahrstreifen aufweisen. Dabei kann der erste Fahrstreifen an die Ausfahrt bzw. einen Verzögerungstreifen, welcher zu der Ausfahrt führt, angrenzen. Der erste Fahrstreifen kann beispielsweise die rechte Spur einer Autobahn sein.

Mittels des Fahrerassistenzsystems wird der Fahrbefehl empfangen, welcher beschreibt, dass das Fahrzeug ausgehend von dem zweiten Fahrsteifen über den ersten Fahrsteifen auf die Ausfahrt der Straße manövriert werden soll. Mit anderen Worten soll also zunächst ein Spurwechsel von dem zweiten Fahrsteifen auf den ersten Fahrstreifen durchgeführt werden und anschließend soll das Fahrzeug die Straße über die Ausfahrt verlassen. Ferner kann das Fahrzeug von der mehrspurigen Straße über die Ausfahrt auf eine andere Straße wechseln. Dies kann beispielsweise bei einem Autobahnkreuz oder dergleichen der Fall sein. Grundsätzlich kann der Fahrbefehl durch eine Routenführung bzw. ein Navigationssystem des Fahrzeugs vorgegeben werden. Alternativ oder zusätzlich kann der Fahrbefehl durch eine entsprechende Bedieneingabe von dem Nutzer begründet sein.

Des Weiteren kann mittels des Fahrerassistenzsystems der Spurwechsel bzw. das Spurwechselmanöver von dem zweiten Fahrstreifen auf den ersten Fahrstreifen vorbereitet werden. Hierzu kann zunächst mit entsprechenden Abstandssensoren bzw. Umfeldsensoren des Fahrerassistenzsystems nach freien Lücken für das Fahrzeug auf dem ersten Fahrstreifen gesucht werden. Darüber hinaus kann mittels des Fahrerassistenzsystems die Geschwindigkeit des Fahrzeugs bzw. die Längsgeschwindigkeit angepasst werden. Insbesondere kann die Geschwindigkeit des eigenen Fahrzeugs verringert werden bzw. an die Geschwindigkeit der weiteren Verkehrsteilnehmer auf dem ersten Fahrstreifen angepasst werden. Beispielsweise kann die Geschwindigkeit des Fahrzeugs derart angepasst werden, dass eine Lücke in der Zielspur verfolgt wird.

Zudem wird der Hinweis an den Nutzer zum Betätigen des Bedienelements bzw. des Blinkerhebels ausgegeben. Infolge der Betätigung des Bedienelements kann dann das Spurwechselmanöver von dem zweiten Fahrstreifen auf den ersten Fahrstreifen ausgelöst bzw. initiiert werden. Das Spurwechselmanöver kann mittels des Fahrerassistenzsystems bzw. eines Spurwechselassistenzsystems automatisch bzw. automatisiert durchgeführt werden. Beim Auslösen des Spurwechselmanövers kann das Spurwechselassistenzsystem zur Durchführung des Spurwechselmanövers getriggert werden.

Gemäß der vorliegenden Erfindung ist es nun vorgesehen, dass die Gesamtreaktionszeit bestimmt wird. Diese Gesamtreaktionszeit beschreibt die Zeitdauer zwischen dem Ausgeben des Hinweises an den Nutzer und dem Auslösen des Spurwechselmanövers. Die Gesamtreaktionszeit ist also diejenige Zeitdauer, die zwischen der Aufforderung des Nutzers, dass dieser das Bedienelement zum Auslösen des Spurwechselmanövers betätigen soll, und dem Auslösen des Spurwechselmanövers durch das Fahrerassistenzsystem verstreicht. Zwischen dem Ausgeben des Hinweises und dem Auslösen des Spurwechselmanövers ist es erforderlich, dass der Nutzer den Hinweis erfasst bzw. wahrnimmt und anschließend das Bedienelement betätigt.

Auf Grundlage dieser Gesamtreaktionszeit wird nun der individuelle Reaktionsfaktor des Nutzers bestimmt. Dieser Reaktionsfaktor beschreibt insbesondere das individuelle bzw. persönliche Reaktionsverhalten des Fahrers. Vorliegend wird berücksichtigt, dass dieser individuelle Reaktionsfaktor für jeden Nutzer individuell ist. Gemäß der vorliegenden Erfindung ist vorgesehen, dass dieser individuelle Reaktionsfaktor für den Nutzer bzw. auch für verschiedene Nutzer im Betrieb des Fahrzeugs bzw. im Laufe der Zeit angelernt wird. Insbesondere soll ein lernendes System bereitgestellt bzw. verwendet werden, welches den individuellen Reaktionsfaktor bestimmen kann.

Mit anderen Worten kann der individuelle Reaktionsfaktor fortlaufend bestimmt werden und bei einem nachfolgenden Manövrieren des Fahrzeugs auf einer mehrspurigen Straße berücksichtigt werden. Unter dem Begriff "nachfolgendes Manövrieren" ist vorliegend insbesondere ein auf das aktuelle Fahrmanöver zeitlich folgendes Fahrmanöver zu verstehen, bei welchem ein Spurwechselmanöver von dem zweiten Fahrstreifen auf den ersten Fahrstreifen vorbereitet wird und hierzu die Geschwindigkeit des eigenen Fahrzeugs angepasst wird. Insgesamt kann somit der Hinweiszeitpunkt und somit die Funktionalität des Fahrerassistenzsystems fortlaufend an den individuellen Nutzer bzw. Fahrer angepasst werden und somit die Akzeptanz für die Funktion verbessert werden.

Gemäß dem Stand der Technik wird der Hinweis an den Nutzer beispielsweise ausgegeben, sobald eine Lücke für das Fahrzeug gefunden wurde. In Abhängigkeit von dem individuellen Reaktionsfaktor kann die Ausgabe des Hinweises früher oder später stattfinden. Bei Nutzern bzw. Fahrern mit einer geringeren Reaktionszeit kann der Hinweis somit früher als standardmäßig vorgesehen ausgegeben werden. Auf diese Weise kann sichergestellt werden, dass das Spurwechselmanöver rechtzeitig durchgeführt wird. Dies ist insbesondere beim Erreichen der Ausfahrt bzw. des der Ausfahrt zugeordneten Verzögerungsstreifens von hoher Relevanz. Insbesondere kann somit sichergestellt werden, dass auch Ausfahrten mit einem kurzen Verzögerungsstreifen und/oder Ausfahrten ohne Verzögerungsstreifen, wie beispielsweise sogenannte tapered exits in den USA, sicher erreicht werden.

Bevorzugt umfasst die gesamte Reaktionszeit eine Nutzerreaktionszeit, eine Nutzerhandlungszeit und eine bekannte Systemzeit. Hierbei kann zum Bestimmen des individuellen Reaktionsfaktors die Nutzerreaktionszeit und die Nutzerhandlungszeit gemessen werden, wobei die Summe aus der Nutzerreaktionszeit und der Handlungsreaktionszeit einer Zeitdauer zwischen dem Ausgeben des Hinweises und dem Betätigen des Bedienelements durch den Nutzer entspricht. Im Betrieb des Fahrerassistenzsystems kann die Zeitdauer zwischen dem Ausgeben des Hinweises mittels des Fahrerassistenzsystems und dem anschließenden Betätigen des Bedienelements bzw. des Blinkerhebels durch den Nutzer gemessen werden. Diese Zeitdauer entspricht der Summe aus der Nutzerreaktionszeit und der Nutzerhandlungszeit. Die Nutzerreaktionszeit kann auch als Reaktionszeit des Nutzers bzw. als mentale Reaktionszeit bezeichnet werden. Die Nutzerreaktionszeit beschreibt die Zeitdauer bzw. die Zeitspanne zwischen dem Erkennen des Hinweises durch den Nutzer und dem Beginn der darauf gerichteten Handlung. Die Nutzerhandlungszeit beschreibt die Zeitdauer, welche der Nutzer zum anschließenden Betätigen des Bedienelements bzw. des Blinkerhebels tatsächlich benötigt. Die Systemzeit, beschreibt diejenige Zeitdauer, welche das Fahrerassistenzsystem zwischen dem Betätigen des Bedienelements durch den Nutzer und dem Auslösen des Spurwechselmanövers benötigt. Diese Systemzeit ist bekannt oder kann in Versuchen ermittelt werden. Somit kann der individuelle Reaktionsfaktor des Nutzers auf Grundlage der Nutzerreaktionszeit, der Nutzerhandlungszeit sowie der bekannten Systemzeit präzise ermittelt werden.

In einer weiteren Ausführungsform wird eine Aufmerksamkeit und/oder eine Müdigkeit des Nutzers bestimmt und der individuelle Reaktionsfaktor wird in Abhängigkeit von der bestimmten Aufmerksamkeit und/oder Müdigkeit bestimmt. Die Müdigkeit und/oder die Aufmerksamkeit des Nutzers bzw. des Fahrers kann mit bekannten Verfahren ermittelt werden. Beispielsweise können hierzu die Daten einer Innenraumkamera und einer nachgeschalteten Bildverarbeitung genutzt werden. Alternativ oder zusätzlich können auch biometrische Sensoren oder dergleichen genutzt werden, um die Aufmerksamkeit und/oder die Müdigkeit des Fahrers ermitteln zu können. Gemäß der vorliegenden Erfindung wird berücksichtigt, dass sowohl die Nutzerreaktionszeit als auch die Nutzerhandlungszeit jeweils individuell für den Nutzer sind, aber auch von der Aufmerksamkeit und/oder Müdigkeit des Nutzers abhängig sind.

Um nun den individuellen Reaktionsfaktor des Nutzers genau bestimmen zu können, wird die Aufmerksamkeit und/oder die Müdigkeit des Nutzers ermittelt und die bestimmte Aufmerksamkeit und/oder Müdigkeit des Nutzers wird bei der Bestimmung des individuellen Reaktionsfaktors auf Grundlage der Nutzerreaktionszeit und der Nutzerhandlungszeit berücksichtigt. Beispielsweise kann für die Müdigkeit und/oder die Aufmerksamkeit des Nutzers ein fester Skalierungsfaktor angenommen werden. Beispielsweise kann eine um 50 % längere Reaktionszeit bei einer Müdigkeit von 100 % festgelegt werden. Auf diese Weise kann der individuelle Reaktionsfaktor mit geringem Rechenaufwand ermittelt werden.

Weiterhin ist vorteilhaft, wenn die Aufmerksamkeit und/oder die Müdigkeit im Betrieb des Fahrzeugs fortlaufend bestimmt wird und der Hinweiszeitpunkt in Abhängigkeit von der bestimmten Aufmerksamkeit und/oder Müdigkeit bestimmt wird. Es kann also zudem vorgesehen sein, dass im Betrieb des Fahrerassistenzsystems bzw. des Fahrzeugs die Müdigkeit und/oder die Aufmerksamkeit des Nutzers fortlaufend bestimmt wird. In Abhängigkeit von der ermittelten Müdigkeit und/oder Aufmerksamkeit kann dann der Hinweiszeitpunkt, an welchem der Hinweis an den Nutzer zum Betätigen des Bedienelements zum Auslösen eines Spurwechselmanövers ausgegeben wird, an die Müdigkeit und/oder Aufmerksamkeit angepasst werden. Beispielsweise kann der Hinweis bei Fahrtantritt bzw. bei einem wachen Nutzer grundsätzlich später ausgegeben werden als bei einem müden Nutzer bzw. einem Nutzer, welcher schon eine gewisse Zeit am Steuer des Fahrzeugs sitzt. Somit kann die Ausgabe des Hinweises bzw. der Hinweiszeitpunkt auch an die aktuelle Aufmerksamkeit und/oder Müdigkeit des Nutzers angepasst werden.

Die Aufmerksamkeit und/oder Müdigkeit des Nutzers kann zudem in Abhängigkeit von der aktuellen Verkehrssituation in der Umgebung des Fahrzeugs bestimmt werden. Insbesondere kann die Aufmerksamkeit des Nutzers in Abhängigkeit von der Verkehrsdichte ermittelt werden. Bei einer geringen Verkehrsdichte kann beispielsweise von einer geringeren Aufmerksamkeit ausgegangen werden als bei dichtem Verkehr bzw. einer hohen Verkehrsdichte.

In einer weiteren Ausgestaltung wird eine Position von zumindest einer Hand des Nutzers bestimmt und bei der Bestimmung des individuellen Reaktionsfaktors auf Grundlage der Nutzerhandlungszeit wird die Position der zumindest einen Hand berücksichtigt. Die Position der zumindest einen Hand des Nutzers kann mit Hilfe einer Innenraumkamera und/oder anhand von Lenkradsensoren ermittelt werden. Vorliegend wird berücksichtigt, dass die Nutzerhandlungszeit, also diejenige Zeitdauer, welcher der Nutzer für das Betätigen des Bedienelements tatsächlich benötigt, von der Position der Hand bzw. der Hände des Nutzers abhängt. Beispielsweise kann die Nutzerhandlungsdauer in dem Fall, in dem der Nutzer die Hand am Lenkrad bzw. in der Nähe des Bedienelements hat, kürzer sein als bei dem Fall, bei welchem mit der Hand ein anderes Bedienelement betätigt wird oder die Hand weit von dem Bedienelement bzw. Blinkerhebel entfernt ist. Somit kann auf Grundlage der Nutzerhandlungszeit der individuelle Reaktionsfaktor des Nutzes auf präzise Weise bestimmt bzw. gelernt werden.

Weiterhin ist vorteilhaft, wenn der individuelle Reaktionsfaktor des Nutzers zusätzlich anhand von Nutzerdaten, welche den Nutzer und/oder ein Fahrverhalten des Nutzers beschreiben, bestimmt wird. Diese Nutzerdaten können im Betrieb des Fahrzeugs durch den Nutzer fortlaufend erfasst und gespeichert werden. Die Nutzerdaten können individuell für den Nutzer bzw. Fahrer gespeichert sein. Beispielsweise können diese Nutzerdaten in einem Schlüssel des Fahrzeugs oder in einem entsprechenden Speicher des Fahrzeugs hinterlegt sein. Diese Nutzerdaten können den Nutzer bzw. Fahrer selbst beschreiben. Beispielsweise können die Nutzerdaten ein Alter des Nutzers beschreiben. somit kann beispielsweise berücksichtigt werden, dass sich die Reaktionszeit des Nutzers mit zunehmendem Alter erhöht. Darüber hinaus können die Nutzerdaten auch ein Fahrverhalten des Nutzers beschreiben. Beispielsweise können die Nutzerdaten beschreiben, ob der Nutzer ein eher sportlicher Fahrer oder ein zurückhaltender Fahrer ist. Zudem können die Nutzerdaten eine Erfahrung des Nutzers beim Betrieb des Fahrzeugs bzw. bei der Nutzung des Fahrerassistenzsystems beschreiben. Unter Berücksichtigung dieser Nutzerdaten kann der individuelle Reaktionsfaktor auf genaue Weise ermittelt werden.

In einer weiteren Ausgestaltung wird der Hinweiszeitpunkt zusätzlich in Abhängigkeit von einer Hinweisart, welche beschreibt, ob der Hinweis optisch, akustisch und/oder haptisch ausgegeben wird, bestimmt. Die Hinweisart bzw. Hinweisform kann also beschreiben, ob der Hinweis zum Betätigen des Bedienelements für die Auslösung des Spurwechselmanövers optisch und/oder akustisch und/oder haptisch ausgegeben wird. Hierbei wird berücksichtigt, dass sich die Reaktionszeiten für visuelle, akustische und haptische Reize sich deutlich unterscheiden können. Damit kann die individuelle Reaktionszeit und somit der Hinweiszeitpunkt präzise gelernt werden.

Ein erfindungsgemäßes Fahrerassistenzsystem bzw. Assistenzsystem für ein Fahrzeug ist zum Durchführen eines erfindungsgemäßen Verfahrens und der vorteilhaften Ausgestaltungen davon eingerichtet. Das Fahrerassistenzsystem kann dazu eingerichtet sein, einen Fahrbefehls zum Manövrieren des Fahrzeugs von einem zweiten Fahrstreifen der Straße über einen ersten Fahrstreifen der Straße auf eine Ausfahrt der Straße zu empfangen. Diesen Fahrbefehl kann das Fahrerassistenzsystem insbesondere von einem Navigationssystem des Fahrzeugs empfangen.

Des Weiteren kann das Fahrerassistenzsystem dazu eingerichtet sein, eine freie Lücke für das Fahrzeug zwischen Verkehrsteilnehmern auf dem ersten Fahrstreifen zu suchen. Das Fahrerassistenzsystem kann zumindest einen Umfeldsensor aufweisen, mittels welchem die weiteren Verkehrsteilnehmer in der Umgebung des Fahrzeugs und insbesondere auf dem benachbarten Fahrstreifen erfasst werden können. Darüber hinaus kann das Fahrerassistenzsystem bzw. eine entsprechende Recheneinrichtung des Fahrerassistenzsystems auf Grundlage der Daten des zumindest einen Umfeldsensors freie Lücken zwischen den weiteren Verkehrsteilnehmern erkennen. Diese Recheneinrichtung kann durch zumindest ein elektronisches Steuergerät des Fahrzeugs gebildet sein. Grundsätzlich kann die Recheneinrichtung zumindest einen Prozessor und einen Speicher aufweisen.

Zudem kann das Fahrerassistenzsystem dazu eingerichtet sein, einen Hinweis an den Nutzer zum Betätigen eines Bedienelements zum Auslösen eines Spurwechselmanövers von dem zweiten Fahrstreifen in die freie Lücke des ersten Fahrstreifens auszugeben, wobei der Hinweis zu einem bestimmten Hinweiszeitpunkt ausgegeben wird. Zum Ausgeben des Hinweises kann das Fahrerassistenzsystem eine Ausgabeeinrichtung aufweisen. Darüber hinaus kann das Fahrerassistenzsystem dazu eingerichtet sein, eine Gesamtreaktionszeit, welche eine Zeitdauer zwischen dem Ausgeben des Hinweises und dem Auslösen des Spurwechselmanövers beschreibt, zu bestimmen. Das Fahrerassistenzsystem kann eine entsprechende Messeinrichtung aufweisen, mittels welcher die Zeitdauer zwischen der Ausgabe des Hinweises und dem Betätigen des Bedienelements bzw. des Blinkerhebels durch den Nutzer erfasst werden kann. Zudem ist das Fahrerassistenzsystem dazu eingerichtet, den individuellen Reaktionsfaktor des Nutzers anhand der Gesamtreaktionszeit zu bestimmen und den Hinweiszeitpunkt für ein nachfolgendes Manövrieren des Fahrzeugs in Abhängigkeit von dem individuellen Reaktionsfaktor anzupassen.

Des Weiteren kann das Fahrerassistenzsystem eine Innenraumsensorik, beispielsweise eine Innenraumkamera, aufweisen, mittels welcher eine Aufmerksamkeit und/oder Müdigkeit des Fahrers ermittelt werden kann. Mittels dieser Innenraumsensorik kann zudem die Position der Hände des Nutzers bestimmt werden.

Darüber hinaus kann mittels des Fahrerassistenzsystems die Längsführung des Fahrzeugs zur Vorbereitung des Spurwechselmanövers angepasst werden. Hierzu können mittels der Recheneinrichtung entsprechende Steuersignale ausgesendet werden. Mittels der Recheneinrichtung bzw. des Fahrerassistenzsystems kann zudem die Trajektorie für das Manövrieren des Fahrzeugs bzw. für das automatisierte Spurwechselmanöver berechnet werden. Außerdem kann die Recheneinrichtung dazu ausgebildet sein, eine Lenkung bzw. ein Lenksystem des Fahrzeugs anzusteuern. Durch diese Ansteuerung der Lenkung kann die Querführung des Fahrzeugs bei dem Spurwechselmanöver übernommen werden. Es kann auch vorgesehen sein, dass mittels des Fahrerassistenzsystems die Längsführung des Fahrzeugs während des Spurwechselmanövers übernommen wird.

Ein erfindungsgemäßes Fahrzeug umfasst ein erfindungsgemäßes Fahrerassistenzsystem. Das Fahrzeug kann insbesondere als Personenkraftwagen ausgebildet sein.

Die mit Bezug auf das erfindungsgemäße Verfahren vorgestellten und bevorzugten Ausführungsformen und deren Vorteile gelten entsprechend für das erfindungsgemäße Fahrerassistenzsystem sowie das erfindungsgemäße Fahrzeug.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen.

Die Erfindung wird nun anhand von bevorzugten Ausführungsbeispielen sowie unter Bezugnahme auf die beigefügten Zeichnungen näher erläutert. Dabei zeigen:

Fig. 1    eine schematische Darstellung eines Fahrzeugs, welches ein Fahrerassistenzsystem zum Manövrieren eines Fahrzeugs auf einer mehrspurigen Straße aufweist; und

Fig. 2    das Fahrzeug gemäß Fig. 1 in einer Verkehrssituation, wobei sich das Fahrzeug auf einer mehrspurigen Straße befindet und ein Fahrmanöver ausgehend von einem zweiten Fahrstreifen der Straße zu einer Ausfahrt der Straße geplant ist.

In den Figuren werden gleiche oder funktionsgleiche Elemente mit den gleichen Bezugszeichen versehen.

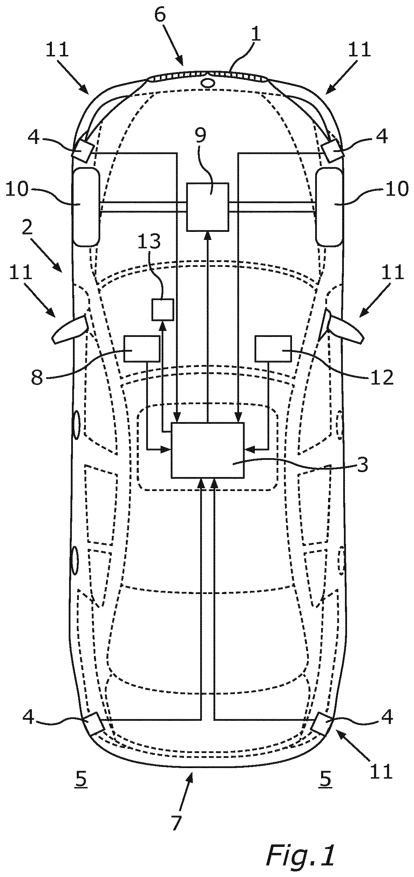

Fig. 1 zeigt ein Fahrzeug 1, welches vorliegend als Personenkraftwagen ausgebildet ist, in einer Draufsicht. Das Fahrzeug 1 umfasst ein Fahrerassistenzsystem 2, mittels welchem ein Nutzer des Fahrzeugs 1 beim Manövrieren des Fahrzeugs 1 auf einer mehrspurigen Straße 15 unterstützt werden kann. Das Fahrerassistenzsystem 2 umfasst eine Recheneinrichtung 3, welche beispielsweise durch zumindest ein elektronisches Steuergerät des Fahrzeugs 1 gebildet sein kann.

Darüber hinaus umfasst das Fahrerassistenzsystem 2 zumindest einen Umfeldsensor 4 bzw. Abstandssensor. In dem vorliegenden Beispiel umfasst das Fahrerassistenzsystem 2 vier Umfeldsensoren 4, von denen zwei Umfeldsensoren 4 in einem Frontbereich 6 und zwei Umfeldsensoren 4 in einem Heckbereich 7 des Fahrzeugs 1 angeordnet sind. In dem gezeigten Beispiel sind die Umfeldsensoren 4 in den jeweiligen Ecken des Fahrzeugs 1 angeordnet. Die Umfeldsensoren 4 können beispielsweise als Radarsensoren ausgebildet sein. Mit den Umfeldsensoren 4 können entsprechende Messungen durchgeführt werden, um Objekte und insbesondere weitere Verkehrsteilnehmer 14 in einer Umgebung 5 des Fahrzeugs 1 erfassen zu können.

Die Recheneinrichtung 3 ist ferner dazu eingerichtet, eine an einem Bedienelement 8 durchgeführte Bedienhandlung von dem Nutzer zu erfassen. Bei dem Bedienelement 8 kann es sich insbesondere um einen Blinkerhebel handeln. Sobald der Blinkerhebel bzw. das Bedienelement 8 von dem Nutzer betätigt wird, können entsprechende Fahrtrichtungsanzeiger 11 des Fahrzeugs 1 aktiviert werden. Des Weiteren umfasst das Fahrerassistenzsystem 2 eine Innenraumsensorik 12, welche beispielsweise eine Innenraumkamera umfassen kann. Mit Hilfe der Innenraumsensorik 12 kann eine Müdigkeit und/oder Aufmerksamkeit des Nutzers bestimmt werden. Ferner ist vorgesehen, dass mittels der Innenraumsensorik 12 eine Position von zumindest einer Hand des Nutzers ermittelt wird. Ferner umfasst das Fahrerassistenzsystem 2 eine Ausgabeeinrichtung 13, mittels welcher ein Hinweis an den Nutzer des Fahrzeugs 1 ausgegeben werden kann.

Darüber hinaus ist die Recheneinrichtung 3 dazu eingerichtet, ein nur vorliegend schematisch dargestelltes Lenksystem 9 des Fahrzeugs 1 anzusteuern. Durch die Ansteuerung des Lenksystems 9 kann die Querführung des Fahrzeugs 1 bei einem Spurwechselmanöver übernommen werden. Dabei können die durch die Ansteuerung des Lenksystems 9 die lenkbaren Räder 10 des Fahrzeugs gelenkt werden. Bevorzugt ist vorgesehen, dass mittels der Recheneinrichtung 3 zudem ein Antriebsmotor und/oder ein Bremssystem des Fahrzeugs 1 angesteuert werden kann, um die Längsführung des Fahrzeugs 1 zu übernehmen.

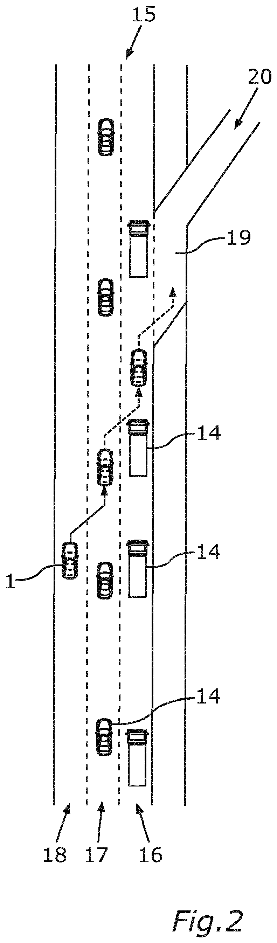

Fig. 2 zeigt in einer schematischen Darstellung eine Verkehrssituation, bei welcher sich das Fahrzeug 1 auf einer mehrspurigen Straße 15 befindet. In dem vorliegenden Beispiel ist die mehrspurige Straße als Autobahn ausgebildet. Die Straße 15 umfasst grundsätzlich zumindest zwei Fahrstreifen 16, 17. In dem vorliegenden Beispiel umfasst die Straße 15 drei Fahrstreifen 16, 17, 18. Vorliegend ist nur eine Richtungsfahrbahn der Straße 15 gezeigt. Dabei befindet sich das Fahrzeug 1 aktuell auf der linken Spur bzw. einem dritten Fahrstreifen 18. Zudem umfasst die Straße 15 einen Verzögerungsstreifen 19. Dieser Verzögerungsstreifen 19 ist einer Ausfahrt 20 zugeordnet.

Vorliegend ist geplant bzw. durch einen Fahrbefehl vorgegeben, dass das Fahrzeug 1 ausgehend von dem dritten Fahrstreifen 18 über den zweiten Fahrstreifen 17, den ersten Fahrstreifen 16 und den Verzögerungsstreifen 19 auf die Ausfahrt 20 manövriert wird. Mittels des Fahrerassistenzsystems 2 wird der Nutzer des Fahrzeugs 1 bei den jeweiligen Spurwechselmanövern unterstützt. Hierzu wird mittels des Fahrerassistenzsystems 2 auf Grundlage der Messungen der Umfeldsensoren 4 nach freien Lücken zwischen den Verkehrsteilnehmern 14 gesucht. Sobald eine freie Lücke zwischen den Verkehrsteilnehmern 14 auf dem zweiten Fahrstreifen 17 erkannt wird, wird eine Geschwindigkeit bzw. Längsgeschwindigkeit des Fahrzeugs angepasst bzw. reduziert. Zudem wird zu einem Hinweiszeitpunkt ein Hinweis an den Fahrer mittels der Ausgabeeinrichtung 13 ausgegeben, dass dieser das Bedienelement 8 betätigen soll und somit den automatisierten Spurwechsel von dem dritten Fahrstreifen 18 auf den zweiten Fahrstreifen 17 auslösen soll. In gleicher Weise werden dann die Spurwechselmanöver von dem zweiten Fahrstreifen 17 auf den ersten Fahrstreifen 16 sowie von dem ersten Fahrstreifen 16 auf den Verzögerungsstreifen 19 vorbereitet und danach durchgeführt.

Vorliegend ist vorgesehen, dass die Ausgabe des Hinweises bzw. der Hinweiszeitpunkt an die individuelle Reaktionszeit des Nutzers angepasst wird. Hierzu kann die Gesamtreaktionszeit $t_{GR}$ gemäß folgender Formel bestimmt werden:

$$t_{GR} = t_{NR}(m\ddot{u}d, indiv) + t_{NH}(m\ddot{u}d, indiv) + t_{sys}.$$

Die Gesamtreaktionszeit $t_{GR}$ beschreibt die Zeitdauer zwischen dem Ausgeben des Hinweises mittels der Ausgabeeinrichtung 13 und dem Auslösen des Spurwechselmanövers durch das Fahrerassistenzsystem 2. Dabei setzt sich die Gesamtreaktionszeit $t_{GR}$ aus der Nutzerreaktionszeit $t_{NR}$, der Nutzerhandlungszeit $t_{NH}$ sowie der Systemzeit $t_{sys}$ des Fahrerassistenzsystems 2 zusammen. Dabei ist sowohl die Nutzerreaktionszeit $t_{NR}$ als auch die Nutzerhandlungszeit $t_{NH}$ einerseits individuell für den Nutzer und andererseits von der Müdigkeit bzw. Aufmerksamkeit des Nutzers abhängig. Dies kann mit folgender Formel beschrieben werden:

$$t_{GR} = t_{NR} \cdot (c_{m\ddot{u}d} + c_{indiv}) + t_{NH} \cdot (c_{m\ddot{u}d} + c_{indiv}) + t_{sys}.$$

Dabei beschreibt der Faktor $c_{m\ddot{u}d}$ die aktuelle Müdigkeit bzw. Aufmerksamkeit des Nutzers. Dieser Faktor kann auf Grundlage der Messungen der Innenraumsensorik 12 bestimmt werden. Der Faktor $c_{m\ddot{u}d}$ kann auch auf Grundlage von weiteren Faktoren bestimmt werden. Beispielsweise kann bei einer geringen Verkehrsdichte in der Umgebung 5 des Fahrzeugs 1 von einer geringeren Aufmerksamkeit ausgegangen werden als bei einer hohen Verkehrsdichte.

Des Weiteren beschreibt $c_{indiv}$ einen individuellen Reaktionsfaktors des Nutzers. Die Nutzerhandlungszeit $t_{NH}$ hängt zudem noch von der Position der Hände des Nutzers ab. Die Position der Hände kann zudem mittels der Innenraumsensorik 12 erfasst werden. In den hier beschriebenen Formeln wird die Position der Hände vorliegend nicht berücksichtigt. Auf Grundlage der Gesamtreaktionszeit $t_{GR}$ kann dann ein individueller Reaktionsfaktor $c_{indiv}$ für den Nutzer ermittelt werden:

$$c_{indiv} = \frac{t_{GR} - c_{m\ddot{u}d}(t_{NR} + t_{NH}) - t_{sys}}{t_{NR} + t_{NH}}.$$

Die Faktoren $c_{m\ddot{u}d}$ und $c_{indiv}$ können bevorzugt auch fortlaufend bestimmt werden und gefiltert bzw. gemittelt werden. Auf Grundlage dieses individuellen Reaktionsfaktors kann dann der Hinweiszeitpunkt für nachfolgende Fahrmanöver mit dem Fahrzeug 1 auf mehrspurigen Straßen 15 angepasst werden. Der individuelle Reaktionsfaktor kann fortlaufend bestimmt bzw. gelernt werden. Dieser kann dann dem Nutzer zugeordnet werden und in einem Nutzerprofil bzw. in einem Fahrzeugschlüssel des Nutzers gespeichert werden.

**Patentansprüche**

1. Verfahren zum Unterstützen eines Nutzers eines Fahrzeugs (1) beim Manövrieren des Fahrzeugs (1) auf einer mehrspurigen Straße (15) mit den Schritten:

- Empfangen eines Fahrbefehls zum Manövrieren des Fahrzeugs (1) von einem zweiten Fahrstreifen (17) der Straße (15) über einen ersten Fahrstreifen (16) der Straße (15) auf eine Ausfahrt (20) der Straße (15),
- Suchen einer freien Lücke für das Fahrzeug (1) zwischen Verkehrsteilnehmern (14) auf dem ersten Fahrstreifen (16),
- Ausgeben eines Hinweises an den Nutzer zum Betätigen eines Bedienelements (8) zum Auslösen eines Spurwechselmanövers von dem zweiten Fahrstreifen (17) in die freie Lücke des ersten Fahrstreifens (16), wobei der Hinweis zu einem bestimmten Hinweiszeitpunkt ausgegeben wird, **gekennzeichnet durch**
- Bestimmen einer Gesamtreaktionszeit, welche eine Zeitdauer zwischen dem Ausgeben des Hinweises und dem Auslösen des Spurwechselmanövers beschreibt,
- Bestimmen eines individuellen Reaktionsfaktors des Nutzers anhand der Gesamtreaktionszeit und

- Anpassen des Hinweiszeitpunkts für ein nachfolgendes Manövrieren des Fahrzeugs (1) in Abhängigkeit von dem individuellen Reaktionsfaktor.

2. Verfahren nach Anspruch 1,
   **dadurch gekennzeichnet, dass**
   die Gesamtreaktionszeit eine Nutzerreaktionszeit, eine Nutzerhandlungszeit und eine bekannte Systemzeit umfasst, wobei zum Bestimmten des individuellen Reaktionsfaktors die Nutzerreaktionszeit und die Nutzerhandlungszeit gemessen werden und wobei die Summe aus der Nutzerreaktionszeit und der Nutzerhandlungszeit einer Zeitdauer zwischen dem Ausgeben des Hinweises und dem Betätigen des Bedienelements (8) durch den Nutzer entspricht.

3. Verfahren nach Anspruch 1 oder 2,
   **dadurch gekennzeichnet, dass**
   eine Aufmerksamkeit und/oder eine Müdigkeit des Nutzers bestimmt wird und der individuelle Reaktionsfaktors in Abhängigkeit von der bestimmten Aufmerksamkeit und/oder Müdigkeit bestimmt wird.

4. Verfahren nach einem der vorhergehenden Ansprüche,
   **dadurch gekennzeichnet, dass**
   die Aufmerksamkeit und/oder die Müdigkeit im Betrieb des Fahrzeugs (1) fortlaufend bestimmt wird und der Hinweiszeitpunkt in Abhängigkeit von der bestimmten Aufmerksamkeit und/oder Müdigkeit bestimmt wird.

5. Verfahren nach einem der Ansprüche 2 bis 4,
   **dadurch gekennzeichnet, dass**
   eine Position von zumindest einer Hand des Nutzers bestimmt wird und bei der Bestimmung des individuellen Reaktionsfaktors auf Grundlage der Nutzerhandlungszeit die Position der zumindest einen Hand berücksichtigt wird.

6. Verfahren nach einem der vorhergehenden Ansprüche,
   **dadurch gekennzeichnet, dass**
   der individuelle Reaktionsfaktor des Nutzers zusätzlich anhand von Nutzerdaten, welche den Nutzer und/oder ein Fahrverhalten des Nutzers beschreiben, bestimmt wird.

7. Verfahren nach einem der vorhergehenden Ansprüche,
   **dadurch gekennzeichnet, dass**
   der Hinweiszeitpunkt zusätzlich in Abhängigkeit von einer Hinweisart, welche beschreibt, ob der Hinweis optisch, akustisch und/oder haptisch ausgegeben wird, bestimmt wird.

8. Fahrerassistenzsystem (2) für ein Fahrzeug (1), wobei das Fahrerassistenzsystem (2) zum Durchführen eines Verfahrens nach einem der vorhergehenden Ansprüche eingerichtet ist.

9. Fahrzeug (1), insbesondere Personenkraftwagen, umfassend ein Fahrerassistenzsystem (2) nach Anspruch 8.

**Claims**

1. Method for assisting a user of a vehicle (1) during the manoeuvring of the vehicle (1) on a multi-lane road (15), having the steps of:

   - receiving a travel command to manoeuvre the vehicle (1) from a second lane (17) of the road (15) across a first lane (16) of the road (15) onto an exit (20) of the road (15),
   - searching for a free gap for the vehicle (1) between road users (14) in the first lane (16),
   - issuing an indication to the user to actuate an operator control element (8) to initiate a lane change manoeuvre from the second lane (17) into the free gap in the first lane (16), wherein the indication is issued at a certain indication time,
   **characterized by**
   - determining a total reaction time which describes a duration between the issuing of the indication and the initiation of the lane change manoeuvre,
   - determining an individual reaction factor of the user based on the total reaction time and
   - adapting the indication time for subsequent manoeuvring of the vehicle (1) on the basis of the individual reaction factor.

**2.** Method according to Claim 1,
**characterized in that**
the total reaction time comprises a user reaction time, a user action time and a known system time, wherein in order to determine the individual reaction factor the user reaction time and the user action time are measured, and wherein the sum of the user reaction time and of the user action time corresponds to a duration between the issuing of the indication and the actuation of the operator control element (8) by the user.

**3.** Method according to Claim 1 or 2,
**characterized in that**
an alertness and/or a tiredness of the user is determined and the individual reaction factor is determined on the basis of the determined alertness and/or tiredness.

**4.** Method according to one of the preceding claims, **characterized in that**
the alertness and/or the tiredness is determined continuously during operation of the vehicle (1) and the indication time is determined on the basis of the determined alertness and/or tiredness.

**5.** Method according to one of Claims 2 to 4, **characterized in that**
a position of at least one hand of the user is determined and the position of the at least one hand is taken into account during the determination of the individual reaction factor based on the user action time.

**6.** Method according to one of the preceding claims, **characterized in that**
the individual reaction factor of the user is additionally determined based on user data which describe the user and/or a driving behaviour of the user.

**7.** Method according to one of the preceding claims, **characterized in that**
the indication time is additionally determined on the basis of an indication type which describes whether the indication is issued visually, audibly and/or haptically.

**8.** Driver assistance system (2) for a vehicle (1), wherein the driver assistance system (2) is set up to carry out a method according to one of the preceding claims.

**9.** Vehicle (1), in particular a passenger car, comprising a driver assistance system (2) according to Claim 8.

**Revendications**

**1.** Procédé pour assister un utilisateur d'un véhicule (1) lors de la manœuvre du véhicule (1) sur une route à plusieurs voies (15), comprenant les étapes suivantes :

- réception d'une instruction de déplacement pour manœuvrer le véhicule (1) depuis une deuxième voie de circulation (17) de la route (15) vers une sortie (20) de la route (15) par le biais d'une première voie de circulation (16) de la route (15),
- recherche d'un espace libre pour le véhicule (1) entre les usagers de la route (14) sur la première voie de circulation (16),
- délivrance d'une notification à l'utilisateur pour actionner un élément de commande (8) afin de déclencher une manœuvre de changement de voie depuis la deuxième voie de circulation (17) dans l'espace libre de la première voie de circulation (16), la notification étant délivrée à un moment de notification déterminé, **caractérisé par**
- détermination d'un temps de réaction total, lequel décrit la durée entre la délivrance de la notification et le déclenchement de la manœuvre de changement de voie,
- détermination d'un facteur de réaction individuel de l'utilisateur à l'aide du temps de réaction total et
- adaptation du moment de notification pour une manœuvre ultérieure du véhicule (1) en fonction du facteur de réaction individuel.

**2.** Procédé selon la revendication 1,
**caractérisé en ce que**
le temps de réaction total comprend un temps de réaction de l'utilisateur, un temps d'action de l'utilisateur et un temps système connu, le temps de réaction de l'utilisateur et le temps d'action de l'utilisateur étant mesurés pour déterminer le facteur de réaction individuel, et la somme du temps de réaction de l'utilisateur et du temps d'action de l'utilisateur correspondant à une durée entre la délivrance de la notification et l'actionnement de l'élément de commande (8) par l'utilisateur.

3. Procédé selon la revendication 1 ou 2,
   **caractérisé en ce que**
   une attention et/ou une fatigue de l'utilisateur est déterminée et le facteur de réaction individuel est déterminé en fonction de l'attention et/ou de la fatigue déterminée.

4. Procédé selon l'une des revendications précédentes, **caractérisé en ce que**
   l'attention et/ou la fatigue est déterminée en continu pendant l'utilisation du véhicule (1) et l'instant de notification est déterminé en fonction de l'attention et/ou de la fatigue déterminée.

5. Procédé selon l'une des revendications 2 à 4, **caractérisé en ce que**
   une position d'au moins une main de l'utilisateur est déterminée et la position de l'au moins une main est prise en compte lors de la détermination du facteur de réaction individuel sur la base du temps d'action de l'utilisateur.

6. Procédé selon l'une des revendications précédentes, **caractérisé en ce que**
   le facteur de réaction individuel de l'utilisateur est déterminé en plus à l'aide de données utilisateur, lesquelles décrivent l'utilisateur et/ou un comportement de conduite de l'utilisateur.

7. Procédé selon l'une des revendications précédentes, **caractérisé en ce que**
   l'instant de notification est en plus déterminé en fonction d'un type de notification, lequel décrit si la notification est émise de manière visuelle, sonore et/ou tactile.

8. Système d'aide à la conduite (2) pour un véhicule (1), le système d'aide à la conduite (2) étant conçu pour mettre en œuvre un procédé selon l'une des revendications précédentes.

9. Véhicule (1), notamment voiture particulière, comprenant un système d'aide à la conduite (2) selon la revendication 8.

*Fig.1*

*Fig.2*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102016216135 A1
- DE 102016216134 A1
- DE 102019008318 A1